# EUROPEAN PATENT APPLICATION

(11) **EP 3 895 755 A1**
(43) Date of publication of application: **20.10.2021**
(21) Application number: 19896862.0
(22) Date of filing: 13.12.2019
(51) Int. Cl.: A61M 39/22

(54) **ANTI-RELEASE CONNECTOR FOR INFUSION LINES**

(30) Priority: 14.12.2018 ES 201831213
(71) Applicant: Servicio Andaluz de Salud, E-41071 Sevilla (ES); Universidad de Sevilla, 41013 Sevilla (ES)
(72) Inventor: MÁRQUEZ RIVAS, Javier, 41071 Sevilla (ES); MAYORGA BUIZA, María José, 41071 Sevilla (ES); GÓMEZ GONZÁLEZ, Emilio, 41013 Sevilla (ES)
(74) Representative: ABG Intellectual Property Law, S.L.
(86) International application number: PCT/ES2019/070847
(87) International publication number: WO 2020/120822

(57) **Abstract**

The invention describes an anti-release connector (1) for infusion lines, which comprises a core (2) from which emerge a main line (3) for connecting an intravenous catheter and two secondary lines (4) for connecting catheters for supplying therapeutic liquids. A handle (5) actuates an obturator contained in the core (2) to selectively connect the main line (3) to the secondary lines (4). The end sections (41) of the secondary lines (4) and the end section (31) of the main line (3) are parallel to one another when viewed perpendicular to a support plane (π) on the surface of the patient's skin. In addition, at least one portion of the end sections (41) forms an angle between 15° and 30° with the end section (31), which is essentially parallel to the support plane (π).

## Description

### OBJECT OF THE INVENTION

The present invention belongs to the field of medicine, and more particularly to the field of infusion systems to infuse a patient with fluids through an intravenous catheter or the like.

The object of the present invention relates to a novel connector, particularly designed to prevent the implanted catheter from being undesirably released and to make handling thereof by medical staff easier.

### BACKGROUND OF THE INVENTION

One of the most common procedures carried out today in any hospital setting involves intravenously infusing a patient with fluids such as, for example, infusing two different fluids, such as saline and medicinal products. At present, this procedure is generally carried out using a device known as a three-way stopcock.

A three-way stopcock is an element provided with two secondary lines that are selectively connected to a main line. The secondary lines are usually arranged on both sides of the main line and form 90° therewith. The main line is connected to an intravenous catheter, the secondary lines are connected to respective sources of therapeutic fluids, and a rotary obturator provided with a handle acting on the core of the stopcock allows selectively communicating a desired secondary line and the main line.

In practice, these stopcocks present various functional problems derived from their configuration *per se.* As mentioned, the three lines in conventional stopcocks emerge from the core according to a radial T-shaped arrangement, i.e., with each line and the adjacent line forming 90° therebetween. The positioning of the main line is fixed by the orientation of the patient's vein to which the intravenous catheter is connected, usually being parallel to the patient's forearm in the direction of the elbow. Accordingly, the two secondary lines are oriented laterally, forming 90° with respect to the main direction of the arm, one on each side. Therefore, catheters or conduits coupled to the two secondary lines emerge from the valve in different directions, and even in opposing directions, to the area where the receptacles containing the liquids to be dispensed are located. Although the length of said catheters theoretically allows them to form curves along their trajectory absorbing such differences in orientation, in practice there are many occasions in which catheters may experience an abrupt bending where the conduit becomes constricted due to the improper movement of the patient, of the support of the receptacles, or to any other cause.

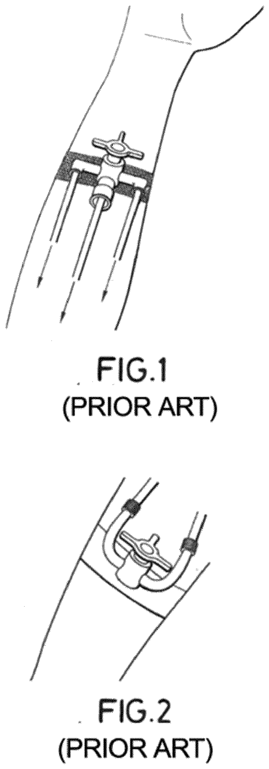

To solve this problem, document ES1052679 describes a three-way stopcock for sanitary use, in which the secondary lines, after emerging from the core of the stopcock, form a 90° bend such that their connection ends are oriented in the same direction as the main line. Therefore, as seen in Figure 1, the catheters which are connected to said secondary lines are naturally arranged in parallel, without requiring curves or abrupt changes in direction.

Document ES1056051, belonging to the same inventor as the preceding document, describes a similar three-way stopcock in which the 90° bend of the secondary lines is replaced with a progressive curve in order to minimize the probability of the bends becoming obstructed. This configuration is described in Figure 2.

These three-way stopcocks in which the ends of the secondary lines are parallel to the main line solve the preceding problems in the sense that the need for the catheters connected to the side lines to form curves along their trajectory is avoided, thereby reducing the probability of constrictions.

However, there still exists a problem relating to the need for handling the stopcock every time the secondary lines are acted on, for example, for connecting or disconnecting the catheter connected to said lines. In these cases, the entire three-way stopcock must be lifted up, removing, to that end, the fixing bandages which keep said stopcock adhered to the patient's skin, and attaching it again once the task in question has been performed. Performing this operation repeatedly takes up a considerable amount of time of the medical professional, in addition to causing discomfort for the patient.

### DESCRIPTION OF THE INVENTION

Based on the configuration of the three-way stopcock described in the mentioned prior art documents, the inventors of the present application solve the preceding problem by means of a novel three-way connector, wherein at least a part of the end sections of the secondary lines, which are parallel to the main line when observed in plan view, form a specific angle with the main line within a plane perpendicular to the plane for fixing to the surface of the patient's skin. In other words, at least a part of the end sections of the secondary lines is oriented *"upwards"* with respect to the surface of the patient's skin. Therefore, the ends of the secondary lines are separated from the surface of the patient's skin, thereby allowing the connection/disconnection of the corresponding catheters without having to remove the connector entirely.

In this document, the terms *"above", "upper", "below", "lower"* and the like are interpreted taking into account the natural orientation of the connector when it is fixed on the surface of a patient's skin. Specifically, it is considered that those elements closest to the patient's skin are located on a *"lower"* side, while those elements farthest away from the patient's skin are located on an *"upper"* side.

The anti-release connector for infusion lines of the present invention therefore comprises a core from which emerge a main line for connecting an intravenous catheter and two secondary lines for connecting catheters for supplying therapeutic liquids. A handle actuates an obturator contained in the core which allows selectively connecting the main line to the secondary lines. In addition, the end sections of the secondary lines and the end section of the main line are parallel to one another when observed in plan view, i.e., according to a direction essentially perpendicular to the support plane of the connector on the surface of the patient's skin. In this context, this means that, regardless of the initial direction of the secondary lines and the end line when they emerge from a core of the connector in which the handle-operated obturator is arranged, their end sections are oriented in parallel and the respective end openings thereof are oriented in the same direction. As mentioned in the prior art section with respect to documents ES1052679 and ES1056051, this allows the catheters connected to the respective lines to be arranged in a parallel manner from the point of connection to said secondary lines, preventing the need for said catheters to follow curved trajectories.

The connector of the present invention clearly differs from the three-way stopcocks of the mentioned prior art documents in that at least one portion of the end sections of the secondary lines forms an angle between 15° and 30°, more preferably from 20° to 25°, with the support plane, which is essentially parallel to the end section of the main line. In other words, taking the support plane corresponding to the lower surface of the connector intended for the fixing thereof to the patient's skin as a reference, the end section of the main line emerging from the core of the connector is essentially parallel to said support plane. This direction makes it easier to insert the needle connected to the corresponding catheter into the patient's venous line. In turn, the end sections of the secondary lines are parallel to said end section of the main line when observed in plan view, i.e., from a point located above the connector according to a direction essentially perpendicular to said support plane. However, within a plane perpendicular to the support plane, at least one portion of said end sections of the secondary lines have the mentioned inclination with respect to the support plane. This configuration will become more apparent based on the attached figures that will be described hereinbelow.

This *"upwards"* inclination of a portion of the end sections of the secondary lines creates a gap between the patient's skin and the openings of the ends of the secondary lines to which the corresponding catheters for the administration of therapeutic liquids are connected. As a result of this gap, a healthcare professional can change the catheter connected to the secondary lines without having to completely remove the connector from the patient's skin.

As mentioned hereinabove, the initial direction of the main lines and the secondary lines in the initial section thereof which emerges from the core of the connector is not relevant provided that the end sections thereof have the mentioned directions. However, in a particularly preferred embodiment of the invention, the main line is entirely straight, and the secondary lines have an initial section which is not parallel, in plan view, to the main line (for example, it may form 90° with the main line) followed by a curved section prior to the corresponding end section parallel to the main line. The curved section may have any curvature, including both 90° bends and smoother curves to prevent obstructions.

According to yet another preferred embodiment of the invention, the connector further comprises a base sheet parallel to the support plane having an upper face fixed to the core and an adhesive lower face intended for being supported on the patient's skin. The base sheet thereby allows fixing the connector of the invention to the surface of the patient's skin in a quick and comfortable manner.

### BRIEF DESCRIPTION OF THE FIGURES

Figure 1 shows a connector according to the prior art.
Figure 2 shows another connector according to the prior art.
Figure 3 shows a perspective view of an example of an anti-release connector according to the present invention.
Figure 4 shows a profile view of the example of an anti-release connector of Figure 3.
Figure 5 shows an elevational view of the example of an anti-release connector of Figure 3.
Figure 6 shows a plan view of the example of an anti-release connector of Figure 3.

### PREFERRED EMBODIMENT OF THE INVENTION

An example of the present invention is described below in reference to the attached figures.

The figures include a reference system (x, y, z) where the direction of the z-axis is the direction perpendicular to the patient's skin when the connector (1) is fixed to the skin according to its natural orientation, the direction of the x-axis is the direction whereby the secondary lines (4) emerge from the core (2), and the direction of the y-axis is the direction whereby the main line (3) emerges from the core. Accordingly, the mentioned π-plane is parallel to the xy-plane.

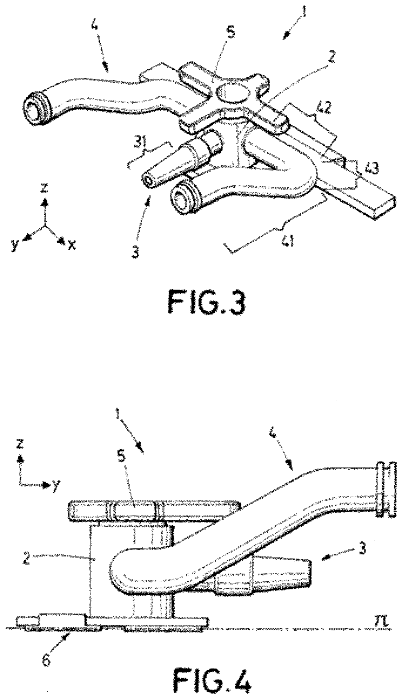

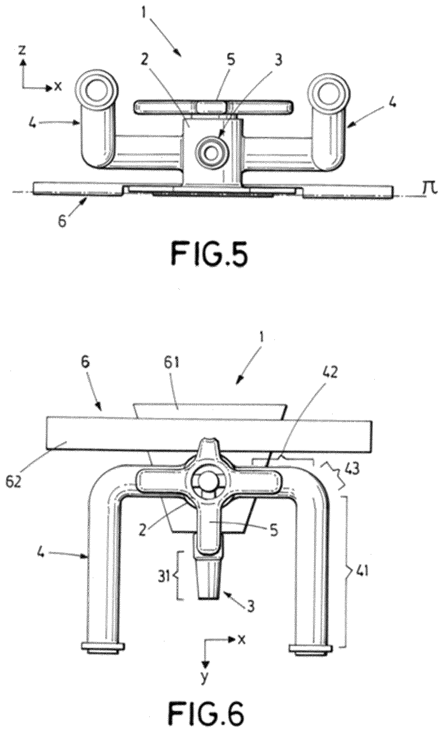

Figures 3-6 show different views of an example of an anti-release connector (1) for infusion lines according to the present invention in which the different parts making up said connector can be seen. The connector (1) is fundamentally formed by a core (2) from which emerge a main line (3) and two secondary lines (4). An obturator (not explicitly shown in the figures) contained inside the core (2) selectively connects the main line (3) to one or both the secondary lines (4). The obturator is turned with the help of a handle (5) that is integral therewith.

In this example, the main line (3) is completely straight, and accordingly both its initial section and its end section (31) are oriented parallel to the y-axis. In turn, the secondary lines (4) have an initial section (42) oriented according to the x-axis, i.e., forming 90° with respect to the main line (3); a curved section (43) corresponding to a 90° turn parallel to the π-plane; and an end section (41) contained in a plane parallel to the yz-plane. Therefore, when observed in plan view, the end section (41) is parallel to the main line (3). Additionally, the end section (41) has a portion forming a 25° angle with respect to the plane (π), and therefore also with respect to the main line (3). Said inclined portion of the end section (41) progressively separates from the support plane (π) and therefore creates a space to make handling the catheters connected to the openings of the ends of the secondary lines (4) easier.

Additionally, the connector (1) of the invention has a base sheet (6) parallel to the plane (π) for fixing thereof to the patient's skin. In this specific example, it can be seen that the base sheet (6) is formed by a trapezoid-shaped support plate (61) rigidly fixed to the lower part of the core (2) of the connector (1) and padded adhesive strips (62) allowing the plate (61) to be adhered to the patient's skin. This specific example uses a padded adhesive strip (62) which is fixed to a portion of the upper face of the support plate (61) and the ends of which project from said plate for fixing same to the patient's skin. Additionally, further padded adhesive strips (62) fixed to the lower face of the support plate (61) can be used. In any case, the configuration adopted by the plate (61) and the padded adhesive strips (62) may vary provided that it allows suitably fixing the connector (1) to the patient's skin.

## Claims

1. An anti-release connector (1) for infusion lines, comprising a core (2) from which emerge a main line (3) for connecting an intravenous catheter and two secondary lines (4) for connecting catheters for supplying therapeutic liquids, wherein a handle (5) which actuates an obturator contained in the core (2) allows selectively connecting the main line (3) to the secondary lines (4), and wherein the end sections (41) of the secondary lines (4) and the end section (31) of the main line (3) are parallel to one another when observed in plan view essentially perpendicular to a support plane (π) on the surface of the patient's skin, **characterized in that** at least one portion of the end sections (41) of the secondary lines (4) forms an angle between 15° and 30° with the support plane (π).

2. The connector (1) according to claim 1, wherein the angle formed by the end sections (41) of the secondary lines (4) with the support plane (π) is between 20° and 25°.

3. The connector (1) according to any of the preceding claims, wherein the main line (3) is straight, and wherein the secondary lines (4) have an initial section (42) which is not parallel, in plan view, to the main line (3) followed by a curved section (43) prior to the corresponding end section (41).

4. The connector (1) according to claim 3, wherein the initial section (42) of the secondary lines (4) forms 90° with the main line (3).

5. The connector (1) according to any of the preceding claims, further comprising a base sheet (6) parallel to the support plane (π) having an upper face fixed to the core (2) and an adhesive lower face intended for being supported on the patient's skin.
